# EUROPÄISCHE PATENTANMELDUNG

(11) **EP 4 375 355 A1**
(43) Veröffentlichungstag der Anmeldung: **29.05.2024**
(21) Anmeldenummer: 22209586.1
(22) Anmeldetag: 25.11.2022
(51) Int. Cl.: C12M 1/00, C12M 1/26

(54) **VORRICHTUNG ZUM BEHANDELN VON ZELLKULTUREN**

(71) Anmelder: Universität Linz, 4040 Linz (AT); Veterinärmedizinische Universität Wien, 1210 Vienna (AT)
(72) Erfinder: Csukovich, Georg, 1030 Wien (AT); Rezk, Marlene, 4175 Herzogsdorf (AT)
(74) Vertreter: Hübscher & Partner Patentanwälte GmbH

(57) **Zusammenfassung**

Es wird eine Vorrichtung zum Behandeln von Zellkulturen mit einem die Zellkulturen aufnehmenden Inkubator (1) beschrieben, der durch Förderleitungen (17, 18) einerseits an eine Versorgungseinrichtung (2) zum Beaufschlagen der Zellkulturen mit einem Medium und anderseits an eine Entsorgungseinrichtung (3) zur Aufnahme des von den Zellkulturen abgepumpten Mediums anschließbar ist. Zur Verbesserung eines Mediumwechsels wird vorgeschlagen, dass der Inkubator (1) einen Stapel (5) aus Zellkulturbehältern (4) mit auf einer Stapelseite parallel ausgerichteten Behälterzugängen (6) sowie einen Kreuzschlitten (7) auf der Zugangsseite des Behälterstapels (5) umfasst, dass der Kreuzschlitten (7) einen zur Ausrichtung der Behälterzugänge (6) parallel verlagerbaren Betätigungskopf (15) mit einer durch die Verlagerung des Betätigungskopfs (15) in die Behälterzugänge (6) einführbaren Kanüle (16) trägt, die wahlweise mit der Förderleitung (17; 18) der Versorgungseinrichtung (2) oder der Entsorgungseinrichtung (3) verbindbar ist, und dass die Förderleitungen (17, 18) eine superhydrophobe Innenoberfläche aufweisen.

## Beschreibung

Die Erfindung bezieht sich auf eine Vorrichtung zum Behandeln von Zellkulturen mit einem die Zellkulturen aufnehmenden Inkubator, der durch Förderleitungen einerseits an eine Versorgungseinrichtung zum Beaufschlagen der Zellkulturen mit einem Medium und anderseits an eine Entsorgungseinrichtung zur Aufnahme des von den Zellkulturen abgepumpten Mediums anschließbar ist.

Zur Züchtung müssen Zellkulturen mit Nähr- und Zusatzstoffen versorgt werden, die den Zellkulturen in Form flüssiger Medien zugeführt werden. Da nach einem Verbrauch der Inhaltsstoffe die Zellkulturen mit frischen, inhaltsreichen Medien beaufschlagt werden müssen, ergibt sich für den Medienwechsel ein erheblicher, sich wiederholender Arbeitsaufwand, weil die die Zellkulturen enthaltenden Zellkulturbehälter aus dem Inkubator entnommen werden müssen, um die verbrauchten Medien aus den Zellkulturbehältern zu entfernen und frische Medien einzufüllen, und zwar unter den erschwerenden Bedingungen der geforderten Sterilität. Um diesen Arbeitsaufwand zu vermeiden, wurde bereits vorgeschlagen (EP 0 447 256 A2), den Inkubator einerseits an eine Versorgungseinrichtung zum Beaufschlagen der Zellkulturen mit einem Medium und anderseits an eine Entsorgungseinrichtung für das von den Zellkulturen abgezogene Medium anzuschließen. Die Versorgungseinrichtung umfasst einen Vorratsbehälter für frisches Medium, das mithilfe einer Förderpumpe durch eine Förderleitung dem Zellkulturbehälter innerhalb des Inkubators zugefördert wird. Zum Abziehen des verbrauchten Mediums ist der Zellkulturbehälter über eine Förderleitung mit der Entsorgungseinrichtung verbunden, die einen Sammelbehälter für verbrauchtes Medium aufweist, das wiederum mithilfe einer Pumpe aus dem Zellkulturbehälter abgezogen wird. Um Kontaminationen der Zellkulturen zu vermeiden, sind die Versorgungseinrichtung ausgangsseitig, die Entsorgungseinrichtung eingangsseitig und der Inkubator ein- und ausgangsseitig mit Sterilisationsfiltern ausgerüstet. Abgesehen von dem durch die Sterilisationsfilter bedingten Konstruktionsaufwand ist als nachteilig anzusehen, dass Vorrichtungen dieser Art nicht für die gleichzeitige Behandlung mehrerer Zellkulturen in einem gemeinsamen Inkubator geeignet sind.

Der Erfindung liegt somit die Aufgabe zugrunde, eine Vorrichtung zur gleichzeitigen Behandlung mehrerer Zellkulturen in gesonderten Zellkulturbehältern unter Einsatz eines gemeinsamen Inkubators so auszugestalten, dass mit vergleichsweise einfachen Konstruktionsmitteln ein steriler Medienaustausch in den einzelnen Zellkulturbehältern sichergestellt werden kann.

Ausgehend von einer Vorrichtung der eingangs geschilderten Art löst die Erfindung die gestellte Aufgabe dadurch, dass der Inkubator einen Stapel aus Zellkulturbehältern mit auf einer Stapelseite parallel ausgerichteten Behälterzugängen sowie einen Kreuzschlitten auf der Zugangsseite des Behälterstapels umfasst, dass der Kreuzschlitten einen zur Ausrichtung der Behälterzugänge parallel verlagerbaren Betätigungskopf mit einer durch die Verlagerung des Betätigungskopfs in die Behälterzugänge einführbaren Kanüle trägt, die wahlweise mit der Förderleitung der Versorgungseinrichtung oder der Entsorgungseinrichtung verbindbar ist, und dass die Förderleitungen eine superhydrophobe Innenoberfläche aufweisen.

Durch die Anordnung eines Stapels aus ausgerichteten Zellkulturbehältern und eines diesen Zellkulturbehältern gegenüber mithilfe eines Kreuzschlittens ausrichtbaren Betätigungskopfs in einem gemeinsamen Inkubator wird es zunächst mit vergleichsweise einfachen technischen Mitteln möglich, die dem Betätigungskopf zugehörige Kanüle bei einer entsprechenden Verlagerung des gegenüber einem ausgewählten Zellkulturbehälters ausgerichteten Betätigungskopfs durch den Behälterzugang in den ausgewählten Zellkulturbehälter als Voraussetzung für einen automatisierten Mediumaustausch einzuführen. Für den Mediumaustausch ist zunächst die in den Zellkulturbehälter eingeführte Kanüle mit der Förderleitung für die Entsorgungseinrichtung zu verbinden, um das Medium aus dem Zellkulturbehälter abpumpen und in die Entsorgungseinrichtung fördern zu können, bevor die Kanüle mit der Förderleitung der Versorgungseinrichtung verbunden und frisches Medium aus der Versorgungsleitung in den Zellkulturbehälter gepumpt wird. Mit dem Ausziehen der Kanüle aus dem Behälterzugang ist der Mediumaustausch für den ausgewählten Zellkulturbehälter abgeschlossen und die Vorrichtung steht für einen Mediumaustausch in einem anderen Zellkulturbehälter bereit.

Da die Förderleitungen zwischen der Versorgungseinrichtung und der Entsorgungseinrichtung einerseits und der Kanüle des Betätigungskopfs anderseits eine superhydrophobe Innenoberfläche aufweisen, können sich in diesen Förderleitungen keine Stoffe ablagern. Unter der üblicherweise geforderten Voraussetzung einer im Bereich der mediumführenden Teile sterilen Versorgungseinrichtung bedeutet dies, dass von einer sterilen Zu- und Abfuhr des von Kontaminationen weitgehend freien Mediums ausgegangen werden kann, sodass sich zusätzliche Sterilisationsfilter erübrigen.

Um die Ausrichtung des Betätigungskopfes gegenüber den Behälterzugängen an unterschiedliche Zellkulturbehälter anpassen zu können, kann der Kreuzschlitten eine Verschiebeführung für den Betätigungskopf aufweisen, die um eine bodenparallele, zur Achse der Kanüle senkrechte Achse schwenkverstellbar gelagert ist.

Da der Inkubator nach der Erfindung im Wesentlichen lediglich mit einem Kreuzschlitten auszurüsten ist und dieser Kreuzschlitten mit dem Betätigungskopf in einfacher Weise zu einer Baueinheit zusammengefasst werden kann, können herkömmliche Inkubatoren ohne besonderen Konstruktionsaufwand erfindungsgemäß umgerüstet werden.

Werden in diesem Zusammenhang die Förderleitungen außerhalb des Inkubators an Förderpumpen angeschlossen, so kann die Baueinheit nicht nur platzsparend ausgeführt, sondern auch in vorteilhafter Weise auf die Bauteile beschränkt werden, die innerhalb des Inkubators für die Mediumförderung in die Zellkulturbehälter und aus den Zellkulturbehältern unverzichtbar sind.

In der Zeichnung ist der Erfindungsgegenstand beispielsweise dargestellt. Es zeigen
- Fig. 1: eine erfindungsgemäße Vorrichtung zum Behandeln von Zellkulturen in einem schematischen Blockschaltbild mit einem in einem horizontalen Schnitt angedeuteten Inkubator und

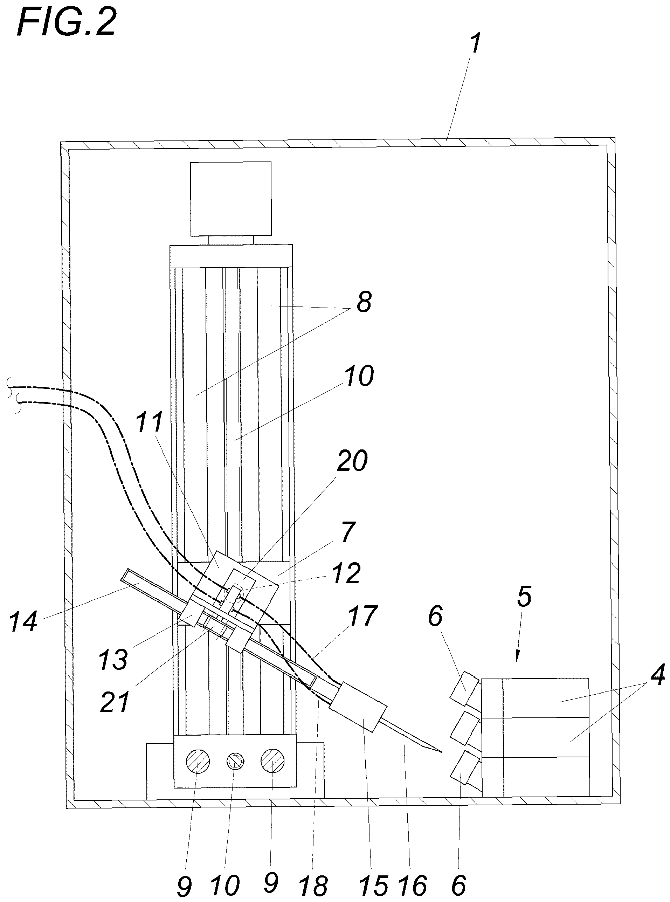

- Fig. 2: den Inkubator nach der Fig. 1 in einem Aufriss in einem größeren Maßstab.

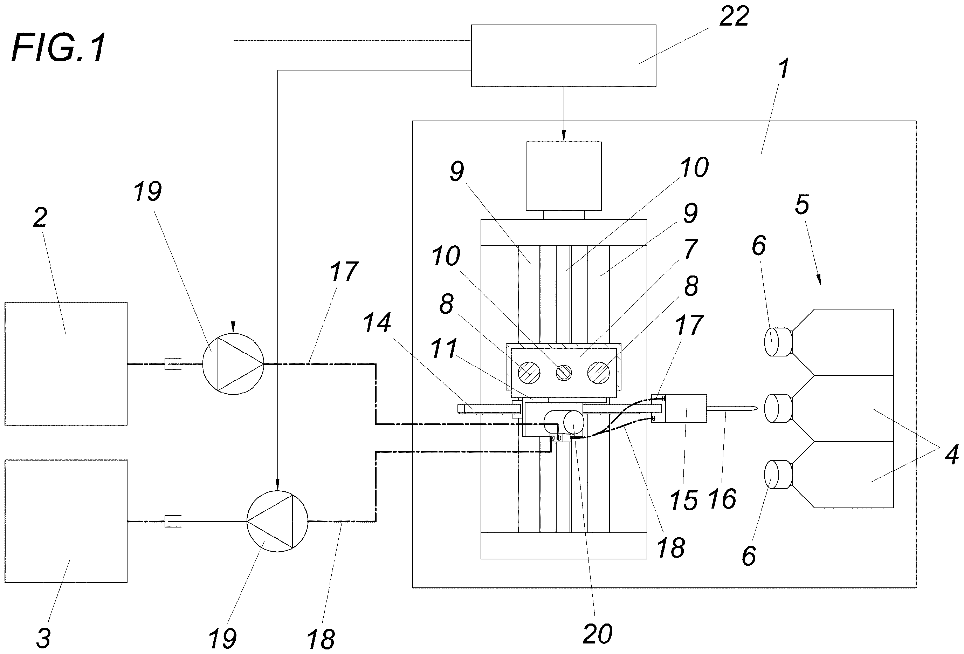

Die Vorrichtung zum Behandeln von Zellkulturen nach der Fig. 1 umfasst einen Inkubator 1, eine Versorgungseinrichtung 2 zum Beaufschlagen der Zellkulturen mit einem Nähr- und Zusatzstoffe enthaltenden, üblicherweise flüssigen Medium und eine Entsorgungseinrichtung 3 zur Aufnahme des von den Zellkulturen abgepumpten Mediums. Die Versorgungseinrichtung 2 weist zu diesem Zweck einen sterilen Vorratsspeicher für das Medium auf, das nach seinem Verbrauch von den Zellkulturen abgezogen und in einen Sammelbehälter der Entsorgungseinrichtung 3 gefördert wird.

Im Inkubator 1 werden mehrere Zellkulturen in gesonderten Zellkulturbehältern 4, üblicherweise Zellkulturflaschen, gleichzeitig behandelt, die parallel ausgerichtet einen in seiner Lage örtlich vorgegebenen Stapel 5 auf einer Seite des Inkubators 1 bilden. Die Ausrichtung der Zellkulturbehälter 4 ist dabei so gewählt, dass ihre in herkömmlicher Art mit einem Verschluss versehenen Behälterzugänge 6 in paralleler Lage gegen das Innere des Inkubators 1 weisen. Auf der dem Stapel 5 gegenüberliegenden Seite des Inkubators 1 ist ein Kreuzschlitten 7 vorgesehen, der der Höhe nach entlang einer Führung 8 verfahrbar ist, die wiederum auf einer bodenparallelen Führung 9 verschiebbar lagert. Als Stelltriebe für den Kreuzschlitten 7 sind Spindeltriebe 10 entlang der Führungen 8 und 9 angedeutet, was jedoch keineswegs zwingend ist.

Auf dem Kreuzschlitten 7 ist ein Schwenklager 11 mit einer zur bodenparallelen Führung 9 parallelen Schwenkachse 12 vorgesehen, das eine Verschiebeführung 13 für einen Arm 14 trägt. An dem dem Stapel 5 zugekehrten Ende des Armes 14 ist ein Betätigungskopf 15 mit einer in Richtung des Armes 14 verlaufenden Kanüle 16 vorgesehen, die beispielsweise über ein Umschaltventil wahlweise an eine von zwei Förderleitungen 17, 18 angeschlossen werden kann. Während die Förderleitung 17 die Kanüle 16 über eine Förderpumpe 19 mit der Versorgungeinrichtung 2 verbindet, dient die Förderleitung 18 zur Strömungsverbindung der Kanüle 16 mit der Entsorgungseinrichtung 3, wobei die Förderleitung 18 ebenfalls mit einer Förderpumpe 19 versehen ist.

Die Kanüle 16 kann mithilfe des Kreuzschlittens 7 und der Schwenklagerung des Armes 14 gegenüber einem ausgewählten Zellkulturbehälter 4 des Stapels 5 ausgerichtet und durch eine Längsverschiebung des Armes 14 durch den jeweiligen Behälterzugang 6 in den Zellkulturbehälter 4 eingeführt werden, um je nach dem Anschluss der Kanüle 16 an die Förderleitung 17 oder 18 entweder Medium aus der Versorgungseinrichtung 2 zuzuführen oder verbrauchtes Medium aus dem Zellkulturbehälter 4 in die Entsorgungseinrichtung 3 abzupumpen. Zum Verlagern des Betätigungskopfes 15 ist ein Stelltrieb vorgesehen, der gemäß dem Ausführungsbeispiel als Zahnstangentrieb mit dem Arm 14 als Zahnstange ausgebildet ist, die mit einem von einem Motor 20 angetriebenen Ritzel 21 kämmt. Andere Stelltriebe sind aber selbstverständlich möglich.

Mithilfe einer Steuereinrichtung 22, die in Abhängigkeit von vorgebbaren Parametern nicht nur die Stelltriebe für den Kreuzschlitten 7 und den Betätigungskopf 15, sondern auch die die Förderpumpen 19 und eine allfällige Umschalteinrichtung für den Anschluss der Kanüle 16 an die beiden Förderleitungen 17, 18 ansteuert, kann der Vorgang eines Mediumaustausches für die einzelnen Zellkulturen in vergleichsweise einfacher Art weitgehend automatisiert werden. Das je nach Verbrauch des Mediums erforderliche Auswechseln des Vorratsspeichers der Versorgungseinrichtung 2 und des Sammelbehälters der Entsorgungseinrichtung für das verbrauchte Medium bedarf ebenfalls keines besonderen Handhabungsaufwands, weil lediglich die Förderleitungen 17, 18 mithilfe entsprechender Leitungskupplungen 23 mit der Versorgungseinrichtung 2 und mit der Entsorgungseinrichtung 3 steril verbunden werden müssen. Wegen der Ausbildung der Förderleitungen 17, 18 mit einer superhydrophoben Innenoberfläche entfallen sonst erforderliche Sterilisationsfilter und damit auch der für diese Filter erforderliche Konstruktions- und Wartungsaufwand. Als superhydrophobe Oberfläche wird dabei eine Oberfläche angesehen, bei der der Kontaktwinkel der Flüssigkeitströpfchen größer als 150° ist.

## Patentansprüche

1. Vorrichtung zum Behandeln von Zellkulturen mit einem die Zellkulturen aufnehmenden Inkubator (1), der durch Förderleitungen (17, 18) einerseits an eine Versorgungseinrichtung (2) zum Beaufschlagen der Zellkulturen mit einem Medium und anderseits an eine Entsorgungseinrichtung (3) zur Aufnahme des von den Zellkulturen abgepumpten Mediums anschließbar ist, **dadurch gekennzeichnet, dass** der Inkubator (1) einen Stapel (5) aus Zellkulturbehältern (4) mit auf einer Stapelseite parallel ausgerichteten Behälterzugängen (6) sowie einen Kreuzschlitten (7) auf der Zugangsseite des Behälterstapels (5) umfasst, dass der Kreuzschlitten (7) einen zur Ausrichtung der Behälterzugänge (6) parallel verlagerbaren Betätigungskopf (15) mit einer durch die Verlagerung des Betätigungskopfs (15) in die Behälterzugänge (6) einführbaren Kanüle (16) trägt, die wahlweise mit der Förderleitung (17; 18) der Versorgungseinrichtung (2) oder der Entsorgungseinrichtung (3) verbindbar ist, und dass die Förderleitungen (17, 18) eine superhydrophobe Innenoberfläche aufweisen.

2. Vorrichtung nach Anspruch 1, **dadurch gekennzeichnet, dass** der Kreuzschlitten (7) eine Verschiebeführung (13) für den Betätigungskopf (15) aufweist, die um eine bodenparallele, zur Achse der Kanüle (16) senkrechte Achse schwenkverstellbar gelagert ist.

3. Vorrichtung nach Anspruch 1 oder 2, **dadurch gekennzeichnet, dass** die Förderleitungen (17, 18) außerhalb des Inkubators (1) an Förderpumpen (19) angeschlossen sind.
